# EUROPÄISCHE PATENTSCHRIFT

(11) **EP 0 983 029 B1**
(45) Veröffentlichungstag und Bekanntmachung des Hinweises auf die Patenterteilung: **01.10.2003**
(21) Anmeldenummer: 98922604.8
(22) Anmeldetag: 14.03.1998
(51) Int. Cl.: A61B 17/16, A61F 2/36

(54) **FRÄSERWERKZEUG ZUM AUSRÄUMEN DES FEMUR-MARKRAUMS UND IN DIESEN RAUM EINZUSETZENDE HÜFTPROTHESE**
CUTTING TOOL FOR CLEANING OUT THE FEMUR MEDULLARY SPACE AND ARTIFICIAL HIP TO BE INSERTED INTO THIS SPACE
OUTIL DE FRAISAGE DESTINE A VIDER LA CAVITE MEDULLAIRE DU FEMUR ET PROTHESE DE LA HANCHE IMPLANTEE DANS CETTE CAVITE

(30) Priorität: 20.03.1997 DE 19711532
(43) Veröffentlichungstag der Anmeldung: 08.03.2000
(73) Patentinhaber: Lubinus, Philipp, 24105 Kiel (DE)
(72) Erfinder: Lubinus, Philipp, 24105 Kiel (DE)
(74) Vertreter: Biehl, Christian, Dipl.-Phys.
(86) Internationale Anmeldenummer: DE9800763
(87) Internationale Veröffentlichungsnummer: WO98042263

(56) Entgegenhaltungen:
- EP-A- 0 359 485
- EP-A- 0 684 018
- WO-A-92/10138
- WO-A-94/27507
- US-A- 4 706 659
- DATABASE WPI Section PQ, Week 8512 Derwent Publications Ltd., London, GB; Class P31, AN 85073545 XP002071666 & SU 1 111 748 A (KIEV MEDICAL) , 7.September 1984

## Beschreibung

Die Erfindung betrifft ein Fräserwerkzeug zum Ausräumen des Femur-Markraums unter Schaffung eines Raums für den Schaft einer Hüftprothese und eine zum Einsetzen in diesen Raum geeignete Hüftprothese.

Das Einsetzen einer aus einem Schaft und einer winklig an diesen angesetzten Kugel bestehenden Hüftprothese ist ein häufiger, regelmäßig relativ unkomplizierter chirurgischer Eingriff.

Schwierigkeiten treten jedoch in den Fällen auf, in denen der vor dem Einbringen des Schafts der Hüftprothese unter Verwendung eines Fräserwerkzeugs auszuräumende Femur-Markraum nicht gestreckt, sondern gekrümmt verläuft, da dann bei der Verwendung der üblichen - gestreckt ausgebildeten - Fräserwerkzeuge regelmäßig in nicht erwünschter Weise auch zu erhaltende Knochensubstanz abgefräst wird.

Aus der DE 29 14 455 ist eine Vorrichtung zur Herstellung der Aushöhlung in einem Knochen zum Einsetzen eines künstlichen Gelenkteils bekannt, bei der zwei der Form der herzustellenden Knochenaushöhlung angepaßte Raspelhälften auf einer flexiblen Drehwerkzeug-Antriebswelle angeordnet sind, wobei die beiden Raspelhälften in Axialrichtung gegeneinander bewegbar angeordnet sind.

Weiter ist in der WO 94/275 ein Fräserwerkzeug zum Ausräumen des Femur-Markraums unter Schaffung eines Raums für den Schaft einer Hüftprothese, mit einer biegsamen Seele und mit einer Mehrzahl von konisch ausgebildeten, mit zentralen Durchbohrungen versehenen, übereinander auf die Seele aufgesetzten, rotationssymmetrischen Frässegmenten offenbart, die eine auf die Seele aufgebrachte Rotationskraft übertragen.

Die merkmale des Oberbegriffs des Anspruchs 7 sind beispielweise aus der EP-A-0 359 485 bekannt.

Der Erfindung liegt die Aufgabe zugrunde, ein Fräserwerkzeug zu schaffen, das es ermöglicht, einen Raum für den Schaft einer Hüftprothese, der dem jeweiligen Verlauf des Femur-Markraums folgt, sowie eine Hüftprothese, die in einen solchen, nicht gestreckten, ausgeräumten Femur-Markraum. eingesetzt werden kann, zu schaffen.

Erfindungsgemäß wird diese Aufgabe durch die Merkmale der Anspruche 1 bzw. 7 gelöst. Die Unteransprüche geben bevorzugte Ausgestaltungen der Erfindung an.

Die Erfindung wird im folgenden anhand einer Zeichnung erläutert. Dabei zeigt:
- Fig. 1: eine schematische Darstellung eines derartigen Fräserwerkzeugs bei nicht-gespannter Seele,
- Fig. 2: eine Fig. 1 entsprechende Darstellung bei gespannter Seele,
- Fig. 3: die Hüftprothese bei nichtgespannter Seele und
- Fig. 4: eine Fig. 3 entsprechende Darstellung bei gespannter Seele.

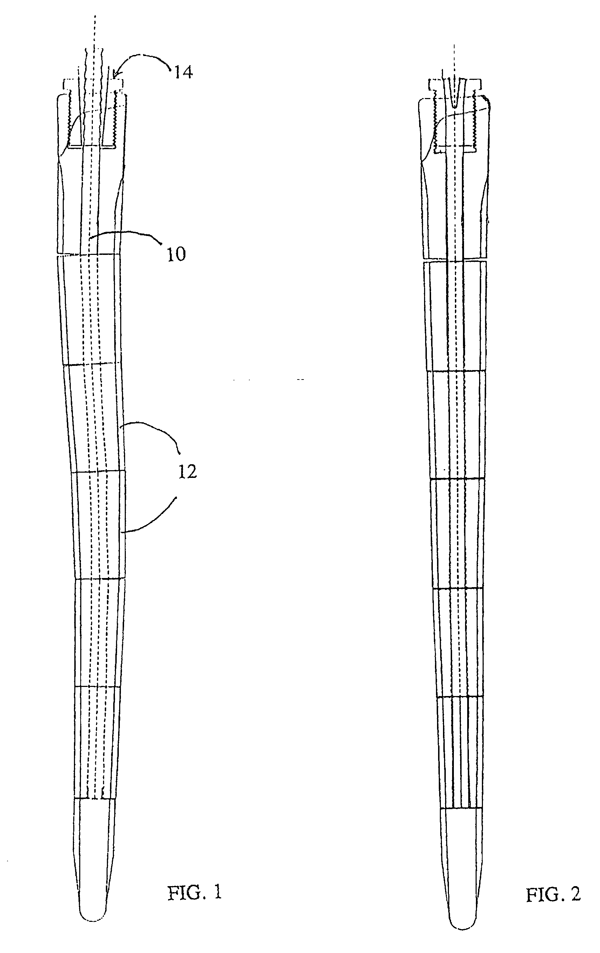

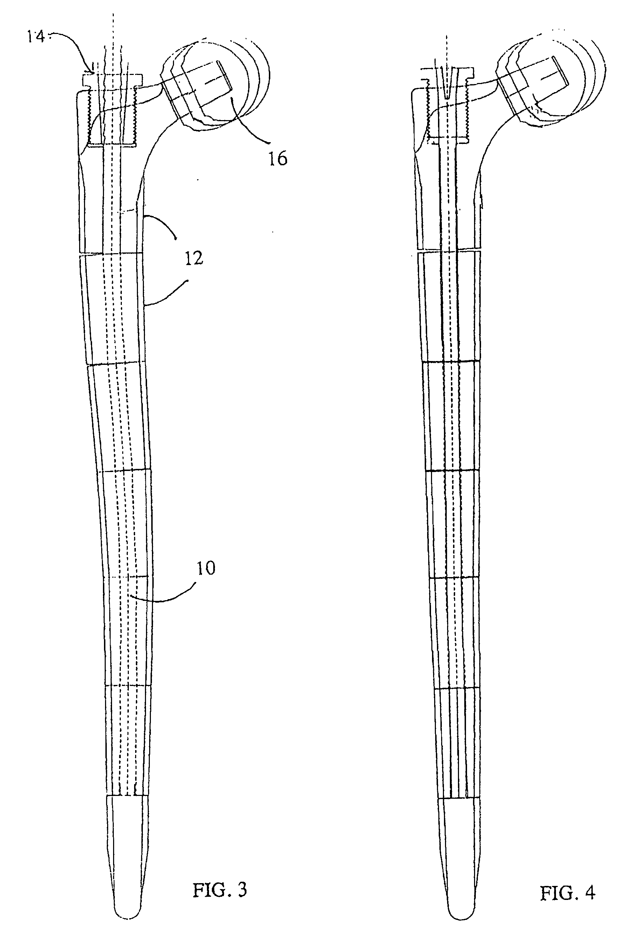

Das in den Figuren 1 und 2 gezeigte Fräserwerkzeug besteht aus einer biegsamen Seele und einer Mehrzahl von mit einer - nicht dargestellten - zentralen Durchbohrung versehenen, übereinander auf die Seele 10 aufgesetzten, jeweils konischen, rotationssymmetrischen Frässegmenten.

Die Seele ist im Querschnitt unrund ausgebildet und die Durchbohrung der Frässegmente 12 mit einer entsprechend unrund axial verlaufenden Durchbohrung versehen (additiv oder alternativ können die zueinander weisenden Stirnflächen der einzelnen Frässegmente 12 auch mit einer ineinander eingreifenden Stirnverzahnung versehen sein).

Die Seele ist mit einer auf das obere der Frässegmente 12 wirkenden Spanneinrichtung 14 versehen, deren Anziehen gegen das obere Frässegment 12 die Seele 10 spannt und dadurch eine Versteifung der Frässegmente 12 gegeneinander (und damit des aus den einzelnen Frässegmenten 12 gebildeten Schafts) bewirkt.

Die Zeichnung deutet weiter an, daß die zueinander weisenden Stirnflächen der Frässegmente leicht konvex ausgebildet sind, so daß diese sich aufeinander abwälzen können.

Das unterste der Frässegmente 12 ist fest an die Seele 10 angesetzt.

Zum Ausräumen des Femur-Markraums wird das Fräserwerkzeug bei nicht festgespannter Seele 10 in üblicher Weise angesetzt. Dabei wird das distale Frässegment 12 (die mit der Seele 10 fest verbundene Spitze) dem Weg des (relativ weichen) Femur-Markraums folgen, die folgenden Frässegmente folgen jeweils diesem Weg, wobei sie bei der Rotation des Fräserwerkzeugs, die durch Aufbringen einer Rotationskraft auf die Seele und/oder das Obere der Frässegmente erfolgt, überwiegend nur das weiche Material des Femur-Markraums ausräumt, den Knochen also schont. Das Fräserwerkzeug "schlängelt" sich damit durch den gekrümmten Femur-Markraum und räumt diesen unter Schonung des Knochenmaterials aus.

Die in den Figuren 3 und 4 gezeigte Hüftprothese ist entsprechend aufgebaut:

Sie besteht aus einer biegsamen Seele 10, einer Mehrzahl von mit einer zentralen Durchbohrung versehenen, übereinander auf die Seele 10 aufgesetzten, konischen, rotationssymmetrischen Segmenten und einer Einrichtung 14 zum eine Versteifung des aus den einzelnen Segmenten 12 gebildeten Schafts bewirkenden Spannen der Seele 10.

Der Schaft der Hüftprothese wird in dem in Fig. 3 gezeigten Zustand, in dem die Seele 10 nicht gespannt ist, in den zuvor ausgeräumten (nicht streng linear verlaufenden) Femur-Markraum eingetrieben. Bei diesem Vorgang werden sich die einzelnen Segmente 12 derart ausrichten (d. h. zu der - gedachten - Achse des Schafts kippen), daß sich der Schaft dem Femmur-Markraum anpaßt.

Nach dem Eintreiben des Schafts wird die Seele 10 des Schafts gespannt, wodurch der Schaft versteift und in Richtung auf eine zunehmende Streckung belastet wird, was - aufgrund der Elastizität des Knochenmaterials - zu einer großflächigen Anlage der den Schaft bildenden Segmente an der erhaltenen Knochensubstanz führt und damit eine erheblich größere Kraftübertragung des Prothesenschafts auf den Femur erlaubt, als sie bei Verwendung eines gestreckten Schafts erreichbar wäre.

## Patentansprüche

1. Fräserwerkzeug zum Ausräumen des Femur-Markraums unter Schaffung eines Raums für den Schaft einer Hüftprothese, mit einer biegsamen Seele (10) und mit einer Mehrzahl von konisch ausgebildeten, mit jeweils einer zentralen Durchbohrung versehenen, übereinander auf die Seele (10) aufgesetzten, rotationssymmetrischen Frässegmenten (12), auf die eine auf die Seele (10) aufgebrachte Rotationskraft übertrag bar ist, **gekennzeichnet durch** eine Einrichtung (14) zum Spannen der Seele (10).

2. Fräserwerkzeug nach Anspruch 1, **dadurch gekennzeichnet, daß** die Seele (10) im Querschnitt unrund ausgebildet ist und die Frässegmente (12) mit einer entsprechend unrund ausgebildeten, axial verlaufenden Durchbohrung versehen sind.

3. Fräserwerkzeug nach einem der vorangehenden Ansprüche, **dadurch gekennzeichnet, daß** die zentrale Durchbohrung im Querschnitt tailliert ausgebildet ist.

4. Fräserwerkzeug nach einem der vorangehenden Ansprüche, **dadurch gekennzeichnet, daß** die Frässegmente (12) beidseitig mit einer Stirnverzahnung versehen sind.

5. Fräserwerkzeug nach einem der vorangehenden Ansprüche, **dadurch gekennzeichnet, daß** die Stirnflächen der Frässegmente (12) leicht konvex ausgebildet sind.

6. Fräserwerkzeug nach einem der vorangehenden Ansprüche, **dadurch gekennzeichnet, daß** das distal angeordnete Frässegment (12) fest an die Seele (10) angesetzt ist.

7. Hüftprothese mit einem konisch verlaufenden Schaft und einer auf das proximale Ende des Schafts aufgesetzten Kugel (16),
**dadurch gekennzeichnet, daß** die Hüftprothese aus einer biegsamen Seele (10), einer Mehrzahl von mit einer zentralen Durchbohrung versehenen, übereinander auf die Seele (10) aufgesetzten, konischen, rotationssymmetrischen Segmenten (12) und einer Einrichtung (14) zum eine Versteifung des aus den einzelnen Segmenten (12) gebildeten Schafts bewirkenden Spannen der Seele (10) besteht.

8. Hüftprothese nach Anspruch 7, **dadurch gekennzeichnet, daß** die zentrale Durchbohrung im Querschnitt tailliert ausgebildet ist.

9. Hüftprothese nach Anspruch 7 oder 8, **dadurch gekennzeichnet, daß** das distal angeordnete Segment (12) fest an die Seele (10) angesetzt ist.

10. Hüftprothese nach einem der Ansprüche 7, 8 oder 9, **dadurch gekennzeichnet**, daβ die Stirnflächen der Segmente (12) leicht konvex ausgebildet sind.

## Claims

1. Milling tool for emptying the femoral medullary space, whilst creating a space for the shaft of a hip prothesis, with a flexible core (1O and a plurality of conical, rotationally symmetrical milling segments (12), mounted in superimposed manner on the core (10) and provided with each a central through-bore, onto which a rotational force applied to the core (10) is transferable, **characterized by** a device (14) for tensioning the core (10).

2. Milling tool according to claim 1, **characterized in that** the cross-section of the core (10) is noncircular and the milling segments (12) are provided with a correspondingly noncircular, axially directed through-bore.

3. Milling tool according to one of the preceding claims, **characterized in that** the central through-bore has a wasp waist cross-section.

4. Milling tool according to one of the preceding claims, **characterized in that** the milling segments (12) are provided on both sides with radial serrations.

5. Milling tool according to one of the preceding claims, **characterized in that** the end faces of the milling segments (12) are slightly convex.

6. Milling tool according to one of the preceding claims, **characterized in that** the distally positioned milling segment (12) is firmly attached to the core (10).

7. Hip prosthesis with a conically directed shaft and a ball (16) mounted on the proximal end of the shaft, **characterized in that** the hip prosthesis comprises a flexible core (10), a plurality of conically, rotationally symmetrical segments (12), mounted in superimposed manner on the core (10) and provided with a central through-bore and a device (14) for tensioning the core (10), which brings about a stiffening of the shaft formed from the individual segments (12).

8. Hip prosthesis according to claim 7, **characterized in that** the central through-bore has a wasp waist cross-section.

9. Hip prosthesis according to claim 7 or 8, **characterized in that** the distally positioned segment (12) is firmly attached to the core (10).

10. Hip prosthesis according to one of the claims 8, 9 or 10, **characterized in that** the end faces of the segments (12) are slightly convex.

## Revendications

1. Outil de fraisage destiné à vider la cavité médullaire du fémur par la création d'une cavité pour la tige d'une prothèse de hanche ayant une âme flexible (10) et une pluralité de segments de fraise (12) à rotation symétrique, de forme conique, chacun pourvu d'un alésage central, posés les uns sur les autres sur l'âme (10), sur lesquels une force de rotation appliquée sur l'âme (10) peut être transmise, **caractérisé par** un dispositif (14) de tension de l'âme (10).

2. Outil de fraisage selon la revendication 1, **caractérisé en ce que** l'âme (10) est de forme ovale en coupe transversale et les segments de fraise (12) sont munis d'un alésage axial de forme ovale correspondante.

3. Outil de fraisage selon l'une quelconque des revendications précédentes, **caractérisé en ce que** l'alésage central est de forme taillée en coupe transversale.

4. Outil de fraisage selon l'une quelconque des revendications précédentes, **caractérisé en ce que** les segments de fraise (12) sont pourvus des deux côtés d'un engrenage frontal.

5. Outil de fraisage selon l'une quelconque des revendications précédentes, **caractérisé en ce que** les surfaces frontales des segments de fraise (12) sont de forme légèrement convexe.

6. Outil de fraisage selon l'une quelconque des revendications précédentes, **caractérisé en ce que** le segment de fraise (12) disposé de manière distale est fermement appliqué contre l'âme (10).

7. Prothèse de hanche ayant une tige conique et une sphère(16) posée sur l'extrémité proximale de la tige, **caractérisée en ce que** la prothèse de hanche est composée d'une âme flexible (10), d'une pluralité de segments de fraise (12) à rotation symétrique, de forme conique, pourvus d'un alésage central, posés les uns sur les autres sur l'âme (10) et d'un dispositif (14) destiné au raidissement de la tige formée des segments individuels (12) provoquant la tension de l'âme (10).

8. Prothèse de hanche selon la revendication 7, **caractérisée en ce que** l'alésage central est de forme taillée en coupe transversale.

9. Prothèse de hanche selon la revendication 7 ou 8, **caractérisée en ce que** le segment (12) disposé de manière distale est fermement appliqué contre l'âme (10).

10. Prothèse de hanche selon l'une quelconque des revendications 7, 8 ou 9, **caractérisée en ce que** les surfaces frontales des segments (12) sont légèrement convexes.
